# Europäisches Patentamt

# European Patent Office

# Office européen des brevets

(11) Veröffentlichungsnummer: **0 208 004**
**B1**

(12) **EUROPÄISCHE PATENTSCHRIFT**

(45) Veröffentlichungstag der Patentschrift:
10.08.88

(21) Anmeldenummer: 85108384.0

(22) Anmeldetag: 05.07.85

(51) Int. Cl.⁴: **A 61 M 25/00**

(54) Verfahren und Vorrichtung zum sterilen Docken von Kunststoffschlauchabschnitten oder dergleichen.

(43) Veröffentlichungstag der Anmeldung:
14.01.87 Patentblatt 87/3

(45) Bekanntmachung des Hinweises auf die Patenterteilung:
10.08.88 Patentblatt 88/32

(84) Benannte Vertragsstaaten:
**BE DE FR GB IT NL SE**

(56) Entgegenhaltungen:
EP-A-0 044 204
EP-A-0 107 271
DE-A-3 210 964
US-A-3 968 195
US-A-4 412 835

(73) Patentinhaber: **NPBI Nederlands Produktielaboratorium voor Bloedtransfusieapparatuur en Infusievloeistoffen B.V., Runde ZZ41, NL- 7881 HM Emmer Compascuum (NL)**

(72) Erfinder: **Leurink, Hendrik Jacob, Torenweg 5, NL- 7873 BP Odoorn (NL)**
Erfinder: **Kuivenhoven, Antonius Johannus Cornelius, Boerhoorn 12, NL- 7812 BX Emmen (NL)**

(74) Vertreter: **Masch, Karl Gerhard, Patentanwälte Andrejewski, Honke & Partner Theaterplatz 3 Postfach 10 02 54, D-4300 Essen 1 (DE)**

EP 0 208 004 B1

## Beschreibung

Die Erfindung betrifft einerseits ein Verfahren zum sterilen Docken von Kunststoffschlauchabschnitten oder dergleichen, die an ihren miteinander zu verbindenden Enden jeweils durch eine quer zur Schlauchachse verlaufende Abquetschschweißnaht verschlossen sind, wobei die beiden zu dockenden Schlauchenden in eine einachsige Lage gebracht sowie stirnseitig mit Hilfe einer zwischengefahrenen Heizeinrichtung aufgeschmolzen und nach Entfernen der Heizeinrichtung axial gegeneinader gedrückt werden, und andererseits eine Vorrichtung zur Durchführung dieses Verfahrens, bestehend aus zwei, relativ zueinander längsverstellbare Halteblöcken und einer senkrecht dazu zwischen die Halteblöcke ein- und ausfahrbaren Heizeinrichtung, wobei die Halteblöcke jeweils eine in Richtung der Längsverstellbarkeit verlaufende Schlauchaufnahme aufweisen und im Bereich der Schlauchaufnahme in zwei Blockhälften geteilt sind.

"Kuststoffschlauchabschnitte oder dergleichen" meint im Rahmen der Erfindung Kunststoffschlauchabschnitte oder ähnlich geformte Kunststoffteile, die hauptsächlich und insbesondere an Kunststoffbeuteln zum Zu- und Abführen von menschlichem Blut, Infusionsflüssigkeit usw. befestigt sind. In weiterem Sinne sind aber aus Schlauchabschnitten bestehende Beutel, beispielsweise zur Aufnahme von menschlichem Blut, Infusionsflüssigkeiten etc. angesprochen.

Die oben erwähnten Maßnahmen sind aus EP-A2-00 44 204 bekannt. Die beiden zu verbindenden Kunststoffschlauchabschnitte werden hier gleichsam überlappend in beide Halteblöcke eingelegt, woraufhin die zwischen die Halteblöcke fahrende, aus einem elektrisch beheizten Messer bestehende Heizeinrichtung die beiden überstehenden freien Schlauchstücke mit den Abquetschschweißnähten abschneidet und die restlichen Schlauchabschnitte durch einen aufschmelzenden Kontakt mit dem beheizten Messer verschlossen hält. Der aufschmelzende Kontakt wird auch noch beim anschließenden Relativverschieben der Halteblöcke beibehalten, mit dem die beiden zu dockenden Schlauchenden in die einachsige Lage gebracht werden. Störend ist zunächst, daß bei jedem Docken Schlauchstücke abgeschnitten und verworfen werden; je nach Länge des Schlauches ist dann die Zahl der möglichen Dockingvorgänge beschränkt. Störend ist aber auch, daß im Zuge der unvermeidbaren Relativbewegungen zwischen den aufgeschmolzenen Schlauchstirnflächen und dem beheizten Messer im Dockingbereich innere, den Strömungswiderstand erhöhende Kunststoffvorsprünge entstehen. Ferner ist beim Zurückziehen der Heizeinrichtung nicht sichergestellt, daß die Schlauchenden geschlossen bleiben bzw. keine Fremdkörper in die Schlauchenden eindringen können. Darüber hinaus bestehen auch beachtliche vorrichtungstechnische Nachteile. Da nicht ausgeschlossen werden kann, daß am beheizten Messer Kunststoffreste verbleiben, muß für Sterilität vor jedem Docken ein neues Messer in die Vorrichtung eingesetzt werden. Ferner ist ein beachtlicher Führungsaufwand erforderlich, die Halteblöcke müssen nicht nur in Querrichtung relativ zur Heizeinrichtung und in Längsrichtung relativ zueinander, sondern auch noch in Querrichtung relativ zueinander bewegbar sein.

Bei einem anderen Verfahren (US-A-39 68 195) ist es zwar auch schon bekannt, zwei Blutbeutelschläuche dadurch zu docken, daß man die Schläuche im Abstand von ihren freien Enden mit Schlauchklemmen abklemmt und anschließend die mit Hilfe eines Bunsenbrenners erweichten freien Enden axial gegeneinander drückt. Das hat aber die Probleme um die oben geschilderten Maßnahmen bisher keiner Lösung zugeführt, weil hier die freien Enden eine nur einmal verwendbare, besonders aufwendige Ausbildung haben, nämlich aus in die Schläuche eingeklebten und an ihren freien Enden durch eine Membran verschlossenen Röhrchen bestehen.

Der Erfindung liegt die Aufgabe zugrunde, im Rahmen der eingangs genannten Maßnahmen auf wesentlich einfachere und zuverlässigere Art und Weise zu einem sterilen Docken zu kommen.

In verfahrensmäßiger Hinsicht löst die Erfindung diese Aufgabe dadurch, daß die beiden Schlauchenden vor sowie bei der Aufschmelzung einerseits mit zueinander parallelen Abquetschschweißnähten im Abstand von der Heizeinrichtung gehalten, andererseits hinter den Abquetschschweißnähten senkrecht zu letzteren zusammen gedrückt werden und dieses Zusammendrücken unmittelbar nach dem axialen Gegeneinanderdrücken beendet wird. Entsprechend ist die vorrichtungsmäßige Lösung dadurch gekennzeichnet, daß zumindest eine der beiden Blockhälften eines jeden Halteblockes in der Schlauchaufnahme mit einer querverlaufenden Schlauchquetschrippe versehen ist.

Die Erfindung geht von der Überlegung bzw. Erkenntnis aus, daß mit Abquetschschweißnähten verschlossene Kunststoffschläuche auch ohne Verschnitt, d.h. unmittelbar miteinander verbunden werden können, und zwar ohne die Stirnflächen der zu verbindenden Schlauchenden berühren zu müssen. Das gelingt überraschenderweise mit Hilfe des zu den Abquetschschweißnähten senkrechten Zusammendrückens, welches bis zum axialen Gegeneinanderdrücken der Schlauchenden das ansonsten selbsttätige Sich-Öffnen der Abquetschschweißnähte verhindert. Dieser mehr oder weniger selbsttätig auftretende Öffnungseffekt wird, aber anschließend ausgenutzt, um den Schlauchkanal zu öffnen, der im Gegensatz zu Verfahren, bei denen die aufgeschmolzenen Schlauchstirnflächen relativ zu einem Messer bewegt werden, von inneren Wülsten praktisch vollkommen frei ist. Das entfallende Abschneiden von Schlauchstücken und das berührungslose Aufschmelzen der Schlauchstirnflächen machen auch den Aufbau der erfindungsgemäßen Vorrichtung besonders einfach. Darüber hinaus ist die Sterilität der Verbindung in jedem Fall gewährleistet.

Für die weitere Ausgestaltung der Erfindung bestehen mehrere Möglichkeiten. So kann es sich für die richtige Positionierung der aneinander anzuschließenden Schlauchenden empfehlen, die Schlauchenden mit Hilfe von angeformten Fixierflanschen zu halten, die ihrerseits noch Positioniernocken und/oder -ausnehmungen aufweisen können. Entsprechend können die Halteblöcke zu Fixierflanschen der Schlauchenden komplementäre Fixierungsausnehmungen aufweisen. Je nach Dimensionierung der zu dockenden Schlauchenden kann es vorkommen, daß die oben beschriebene Öffnungswirkung aufgrund der Schlauchelastizität nicht ausreicht, um den Schlauchkanal durchgängig zu bekommen. Hier empfiehlt es sich dann, die axial gegeneinander gegeneinander gedrückten Schlauchenden unmittelbar nach Beendigung des zu den Abquetschschweißnähten senkrechten Zusammendrückens in Richtung der Abquetschschweißnähte offengedrückt werden. Entsprechend sollte die Vorrichtung zwischen die Halteblöcke einfahrbare Druckfinger zum Aufdrücken der gedockten Schlauchenden gekennzeichnet sein.

(Aus der EP-A-194 873, die einen Stand der Technik nach Art. 54 (3) EPÜ darstellt, ist ein Verfahren zum sterilen Docken von an ihren miteinander zu verbindenden Enden verschlossenen Kunststoffschlauchabschnitten bekannt, wobei die beiden zu dockenden Schlauchenden mit Abstand voneinander in eine einachsige Lage gebracht werden, danach im Abstand von ihren freien Enden senkrecht zu ihrer Achse in derselben Richtung zusammengedrückt werden, anschließend stirnseitig mit Hilfe einer zwischengefahrenen und dann einen Abstand von den beiden Schlauchenden einhaltenden Heizeinrichtung aufgeschmolzen werden und nach Entfernen der Heizeinrichtung axial gegeneinander gedrückt werden und unmittelbar danach das Zusammendrücken beendet wird.

Von diese Stand der Technik unterscheidet sich das erfindungsgemäße Verfahren dadurch, daß die beiden zu dockenden Schlauchenden jeweils durch eine quer zur Schlauchachse verlaufende Abquetschschweißnaht verschlossen sind und nach dem zu den zueinander parallelen Abquetschschweißnähten senkrechten Zusammendrücken unmittelbar aufgeschmolzen werden.

Im folgenden wird die Erfindung anhand einer Zeichnung erläutert. Es zeigen

Fig. 1 in perspektivischer Darstellung eine Vorrichtung zur Durchführung des erfindungsgemäßen Verfahrens, und

Fig. 2 in gegenüber der Fig. 1 vereinfachter Darstellung die Vorrichtung in verschiedenen aufeinanderfolgenden Verfahrensstufen.

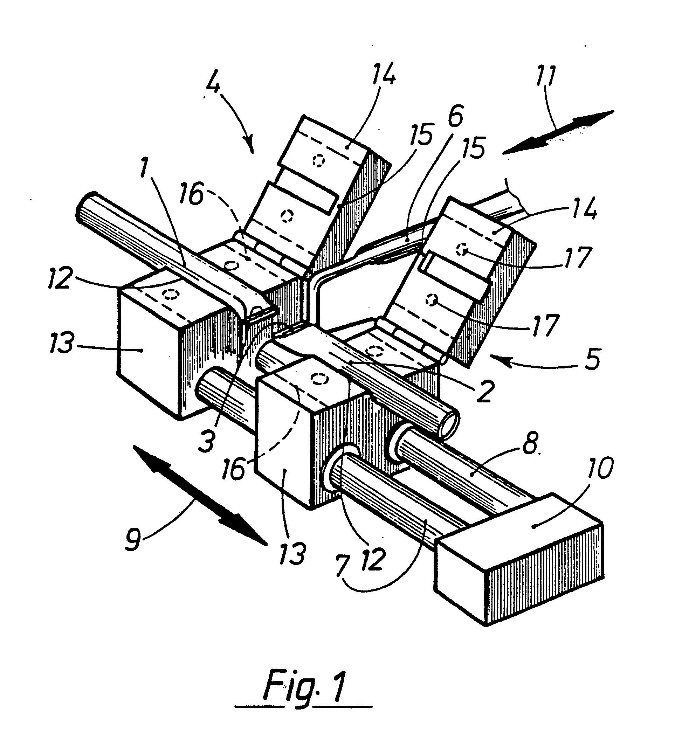

Die Vorrichtung dient zum sterilen Docken von Kunststoffschlauchabschnitten 1, 2, die beispielsweise mit Blutbeuteln in Verbindung stehen und an ihren miteinander zu verbindenden Enden jeweils durch eine quer zur Schlauchachse verlaufende Abquetschschweißnaht 3 verschlossen sind. Die Vorrichtung besteht aus zwei Halteblöcken 4, 5 und einer Heizeinrichtung 6. Die beiden Halteblöcke 4, 5 sind relativ zueinander längsverstellbar; hierzu ist der in Fig. 1 linke Halteblock 4 mit zwei Führungsstangen 7, 8 versehen, auf dem der andere Halteblock 5 in Richtung des Doppelpfeiles 9 längsverschiebbar ist. Das Maß der Längsverschiebbarkeit ist durch einen Anschlag 10 beschränkt, der die freien Enden der Führungsstangen 7, 8 miteinander verbindet. Die Heizeinrichtung 6 besteht aus einem elektrisch beheizten Plattenteil, das in Richtung des weiteren Doppelpfeiles 11 senkrecht zwischen die Halteblöcke 4, 5 ein-und ausfahrbar ist. Die Halteblöcke 4, 5 weisen jeweils eine in Richtung der Längsverstellbarkeit (Doppelpfeil 9) verlaufende Schlauchaufnahme 12 auf, in deren Bereich sie in zwei auseinanderklappbare Blockhälften 13, 14 geteilt sind. Die Blockhälften 13, 14 sind in der Schlauchaufnahme 12 jeweils mit einer querverlaufenden Schlauchquetschrippe 15 versehen. In Fig. 1 ist noch schematisch angedeutet, daß die Halteblöcke 4, 5 zu Fixierflanschen der Schlauchenden komplementäre Fixierungsausnehmungen 16 mit Fixierungsnocken 17 aufweisen können. Nicht mehr dargestellt sind gegebenenfalls zusätzlich zu verwirklichende, zwischen die Halteblöcke 4, 5 einfahrbare Druckfinger zum Aufdrücken der gedockten Schlauchenden, da letzteres ohne weiteres auch von Hand besorgt werden kann.

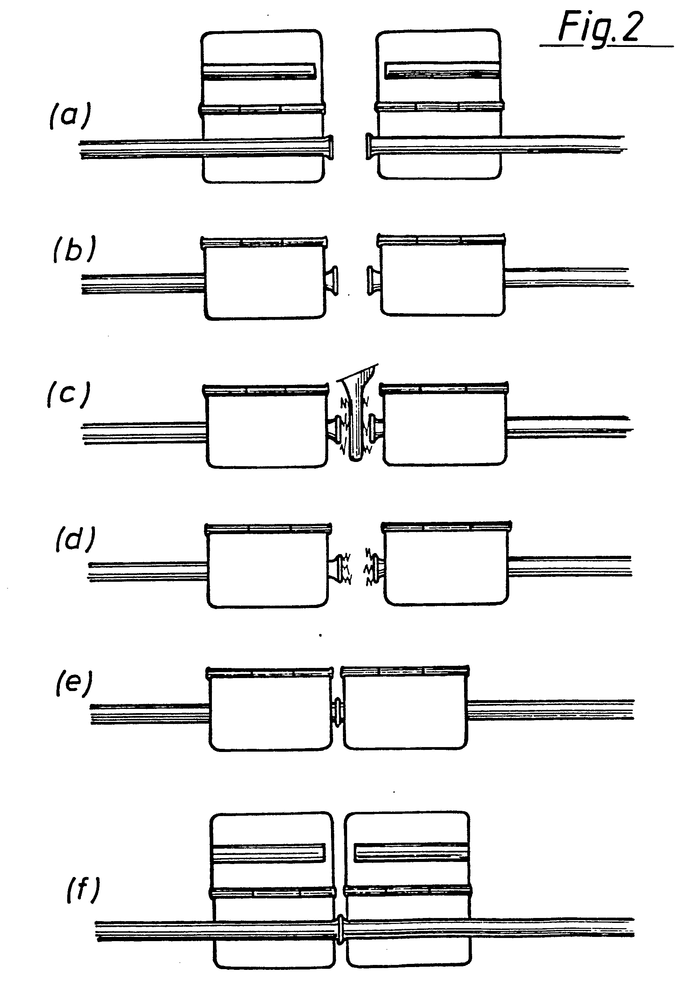

Zum Docken zweier Kunststoffschläuche werden die miteinander zu verbindenden Schlauchenden mit zueinander parallelen Abquetschschweißnähten 3 in geöffneten Halteblöcke 4, 5 eingelegt, wie in Fig. 2 dargestellt wird, und dadurch in eine einachsige Lage gebracht (a). Anschließend werden die Halteblöcke 4, 5 geschlossen (b), wobei die Schlauchquetschrippen 15 die Schlauchenden hinter den Abquetschschweißnähten 3 senkrecht zu letzteren zusammendrücken. Die beiden Abquetschschweißnähte 3 bzw. Halteblöcke 4, 5 weisen einen solchen Abstand voneinander auf, daß nunmehr die Heizeinrichtung 6 mittig zwischenfahren kann, ohne die Schlauchenden zu berühren (c). Das stirnseitige Aufschmelzen der Schlauchenden erfolgt somit nur durch Wärmestrahlung und -konvektion. Nach dem Aufschmelzen wird die Heizeinrichtung 6 wieder zurückgezogen (d) und durch Zusammenfahren der beiden Halteblöcke 4, 5 werden die beiden Schlauchenden axial gegeneinander gedrückt (e). Unmittelbar anschließend, d.h. zu einem Zeitpunkt, wo die Stirnflächen bereits aneinanderhaften, der aufgeschmolzene Kunststoff aber noch nicht wieder erstarrt ist, werden die Halteblöcke 4, 5 geöffnet, so daß sich die Abquetschschweißnähte zufolge der Schlauchelastizität selbsttätig öffnen. Gegebenenfalls wird hierfür durch entsprechendes Drücken mit zwei Fingern nachgeholfen.

Zum Nachweis der Sterilität wurden mehrere Male zwei gammasterilisierte PVC-Schläuche außen um die Abquetschschweißnaht herum mit Mikroflora von menschlichen Händen und Spuren von Bacillus stearothermophillus aus einer Auflösung mit einer Konzentration von $4 \times 10^6$ Spuren / ml verseucht. Nach dem Docken wurden die Schläuche mit Wachstumsmedium TSB ausgespült, das nach einer Inkubationszeit von 7 Tagen auf Wachstum kontrolliert wurde. Ein Bakterienwachstum konnte in keinem Fall festgestellt werden.

**Patentansprüche**

1. Verfahren zum sterilen Docken von Kunststoffschlauchabschnitten (1, 2) oder dergleichen, die an ihren miteinander zu verbindenden Enden jeweils durch eine quer zur Schlauchachse verlaufende Abquetschschweißnaht (3) verschlossen sind, wobei die beiden zu dockenden Schlauchenden in eine einachsige Lage gebracht sowie stirnseitig mit Hilfe einer zwischengefahrenen Heizeinrichtung (6) aufgeschmolzen und nach Entfernen der Heizeinrichtung axial gegeneinander gedrückt werden, dadurch gekennzeichnet, daß die beiden Schlauchenden vor sowie bei der Aufschmelzung einerseits mit zueinander parallelen Abquetschschweißnähten (3) im Abstand von der Heizeinrichtung (6) gehalten, andererseits hinter den Abquetschschweißnähten (3) senkrecht zu letzteren zusammengedrückt werden und dieses Zusammendrücken unmittelbar nach dem axialen Gegeneinanderdrücken beendet wird.

2. Verfahren nach Anspruch 1, dadurch gekennzeichnet, daß die Schlauchenden mit Hilfe von angeformten Fixierflanschen gehalten werden.

3. Verfahren nach Anspruch 1 oder 2, dadurch gekennzeichnet, daß die axial gegeneinander gedrückten Schlauchenden unmittelbar nach Beendigung des zu den Abquetschschweißnähten (3) senkrechten Zusammendrückens in Richtung der Abquetschschweißnähte offen gedrückt werden.

4. Vorrichtung zur Durchführung des Verfahrens nach einem der Ansprüche 1 bis 3, bestehend aus zwei relativ zueinander längsverstellbaren Halteblöcken (4, 5) und einer senkrecht dazu zwischen die Halteblöcke ein- und ausfahrbaren Heizeinrichtung (6), wobei die Halteblöcke (4, 5) jeweils eine in Richtung der Längsverstellbarkeit verlaufende Schlauchaufnahme (1, 2) aufweisen und im Bereich der Schlauchaufnahme in zwei Blockhälften (13, 14) geteilt sind, dadurch gekennzeichnet, daß zumindest eine der beiden Blockhälften (13, 14) eines jeden Halteblockes (4 bzw. 5) in der Schlauchaufnahme (12) mit einer querverlaufenden Schlauchquetschrippe (15) versehen ist.

5. Vorrichtung nach Anspruch 4, dadurch gekennzeichnet, daß die Halteblöcke (4, 5) zu Fixierflanschen der Schlauchenden komplementäre Fixierungsausnehmungen (16) aufweisen.

6. Vorrichtung nach Anspruch 4 oder 5, gekennzeichnet durch zwischen die Halteblöcke (4, 5) einfahrbare Druckfinger zum Aufdrücken der gedockten Schlauchenden.

**Claims**

1. A process for the sterile coupling of lengths of plastics tube (1, 2) or the like, which at each of their ends that are to be coupled together are closed by a squeeze-welded seam (3) running transversely to the axis of the tube, in which the two ends of the tube that are to be coupled are placed in uniaxial position and are melted at their end faces by means of a heating appliance (6) moved between them and after removal of the heating appliance are pressed axially against one another, characterized in that the two tube ends both before and during melting are on one hand held with their squeeze-welded seams (3) parallel to one another at a distance from the heating appliance (6), and on the other hand are pressed together behind their squeeze-welded seams (3) and transversely to the latter, this pressure being ended immediately after the axial pressure of the tube ends against one another.

2. A process according to Claim 1, characterized in that the tube ends are held by means of formed-on fixation flanges.

3. A process according to Claim 1 or 2, characterized in that the tube ends axially pressed against one another are pressed open in the direction of the squeeze-welded seams immediately after ending the pressing-together transversely to the squeeze-welded seams (3).

4. Apparatus for effecting the process according to one of Claims 1 to 3, consisting of two holding blocks (4, 5) longitudinally adjustable relatively to one another, and a heating appliance (6) that is movable in and out transversely between the holding blocks, in which each of the holding blocks (4, 5) possesses a tube receptor (12) extending in the direction of the longitudinal adjustment and is divided into two half blocks (13, 14) at the position of the tube receptor, characterized in that at least one of the two half blocks (13, 14) of each holding block (4 or 5) is provided with a transverse tube-squeezing rib (15) in its tube receptor (12).

5. Apparatus according to Claim 4, characterized in that the holding blocks (4, 5) possess fixing recesses (16) complementary to fixing flanges of the ends of the tubes.

6. Apparatus according to Claim 4 or 5, characterized by pressure fingers that are movable in between the holding blocks (4, 5) to press against the coupled ends of the tubes.

**Revendications**

1. Procédé pour raccorder de façon stérile des éléments de tuyaux en matière plastique (1, 2) ou analogues, qui sont fermés respectivement par une ligne de soudage (3) obtenue par pincement et s'étendant transversalement par rapport à l'axe des tuyaux, et selon lequel on amène les deux extrémités de tuyaux devant être réunies de manière qu'elles soient alignées suivant un axe et on les fait fondre au niveau de leurs faces frontales, à l'aide d'un dispositif de chauffage (6) introduit en position intercalée et on les repousse axialement l'une contre l'autre après retrait du dispositif de chauffage, caractérisé en ce que d'une part on maintient les deux extrémités de tuyaux avant et lors de la fusion de manière que les lignes de soudage (3) obtenues par pincement et parallèles entre elles soient maintenues à distance du dispositif de chauffage (6), et que d'autre part on comprime lesdites extrémités en arrière des lignes de soudage (3) obtenues par pincement, perpendiculairement à ces lignes et que l'on interrompt cette compression directement après avoir repoussé les extrémités des tuyaux axialement l'une contre l'autre.

2. Procédé selon la revendication 1, caractérisé en ce que l'on maintient les extrémités des tuyaux à l'aide de brides de fixation formées par façonnage.

3. Procédé selon la revendication 1 ou 2, caractérisé en ce qu'on repousse les extrémités des tuyaux, repoussées axialement l'une contre l'autre, à l'état ouvert, en direction des lignes de soudage (3) obtenues par pincement, dès la fin de la compression réalisée perpendiculairement aux lignes de soudage obtenues par pincement.

4. Dispositif pour la mise en oeuvre du procédé selon l'une des revendications 1 à 3, constitué par deux blocs de maintien (4, 5) déplaçables longitudinalement l'un par rapport à l'autre et par un dispositif de chauffage (6) pouvant être introduit et ressorti perpendiculairement auxdits blocs et entre ces derniers, les blocs de maintien (4, 5) comportant chacun un logement (1, 2) pour tuyau s'étendant dans la direction de la possibilité de déplacement longitudinal et étant subdivisés en deux demi-blocs (13, 14) dans la zone du logement pour tuyau, caractérisé en ce qu'au moins l'un des deux demi-blocs (13, 14) de chaque bloc de maintien (4 ou 5) est muni, au niveau du logement (1, 2) pour tuyau, d'une nervure transversale (15) de pincement du tuyau.

5. Dispositif selon la revendication 4, caractérisé en ce que les blocs de maintien (4, 5) comportent des évidements de fixation (16), qui sont complémentaires des brides de fixation des extrémités de tuyaux.

6. Dispositif selon la revendication 4 ou 5, caractérisé par des broches de compression pouvant être introduites entre les blocs de maintien (4, 5) et qui servent à comprimer les extrémités de tuyaux réunies.

Fig. 1

Fig.2

(a)

(b)

(c)

(d)

(e)

(f)